# EUROPEAN PATENT SPECIFICATION

(11) **EP 3 562 783 B1**
(45) Date of publication and mention of the grant of the patent: **24.03.2021**
(21) Application number: 17832786.2
(22) Date of filing: 29.12.2017
(51) Int. Cl.: C01C 1/12, B01J 2/00, C01C 1/242, C05C 1/02, C05C 3/00, C07C 273/16

(54) **PROCESSING OF EXHAUST GASES FROM A UREA PLANT**
VERARBEITUNG VON ABGASEN AUS EINER HARNSTOFFANLAGE
TRAITEMENT DE GAZ D'ÉCHAPPEMENT PROVENANT D'UNE INSTALLATION DE PRODUCTION D'URÉE

(30) Priority: 30.12.2016 EP 16207558
(43) Date of publication of application: 06.11.2019
(73) Proprietor: Yara International ASA, 0277 Oslo (NO)
(72) Inventor: VOLKE, Howard, 4541 HJ Sluiskil (NL)
(74) Representative: AWA Sweden AB
(86) International application number: PCT/EP2017/084815
(87) International publication number: WO 2018/122377

(56) References cited:
- WO-A1-2011/032786
- WO-A1-2014/188371
- US-A- 3 928 015

## Description

### TECHNICAL FlELD

The present invention relates to the field of preparing urea ammonium sulphate fertilizers, and more specifically, to the field of processing exhaust gases from a urea plant, the exhaust gas comprising urea dust and ammonia.

### INTRODUCTION

Urea dust and ammonia are known to be present in exhaust gases from urea plants, urea granulation towers, urea prilling towers and chemical fertilizer plants. Such plants in particular release waste air streams that contain dust and ammonia resulting from various process steps. This air stream must be purified before being passed into the environment or recycled back into the process. Such waste air stream results in particular from granulation, prilling and product cooling process steps in a urea production process.

JP 9 227 493 describes a method for recovery of urea dust and ammonia from such a gas stream by contacting said gas stream with an aqueous sulphuric acid solution, thus forming an acid solution of ammonium sulphate and urea. A disadvantage of the known process is, that the resulting product is a solution, which can hardly be used as a fertilizer due to its high transport costs.

As described in DE 10 133 935, it is a desire to add sulphur to nitrogen containing fertilizers. It is a further desire to reduce pollution from industrial activities and in particular from fertilizer production plants.

To this object, WO 2011/032786 provides a method for recovery of urea dust and ammonia from a gas stream by contacting said gas stream with an aqueous sulphuric acid solution, thus forming an acid solution of ammonium sulphate and urea, characterized in that the acid solution is concentrated to a melt comprising less than 5 wt.% of water, which melt is subsequently transferred into solid particles comprising urea and ammonium sulphate.

WO 2014/188371 discloses a method of recovering ammonium sulphate from a gas stream produced in a urea plant, and in particular by a solidification unit of the urea plant, the method including the steps of: treating a gas stream from a solidification unit in a scrubber to remove ammonia from the gas stream and form an ammonium sulphate solution; mixing the ammonium sulphate solution from the scrubber with a low-concentration urea solution produced in the urea plant, and removing water in an evaporator, to form a urea-ammonium sulphate eutectic; and feeding the eutectic, mixed with high-concentration urea melt produced by the urea plant, to the solidification unit. Such a method has several distinct limitations. I.e., said method mixes urea with urea-ammonium sulphate and is therefore limited to the ratio in which sulphur and nitrogen are provided in the finished fertilizer. Also, said method does not provide a homogeneous mixture of urea and ammonium sulphate but provides a heterogeneous urea and ammonium sulphate blend.

Nowadays, the need for fertilizing a soil can be determined accurately, and providing a fertilizer with a very specific sulphur (S) to nitrogen (N) ratio (S/N ratio) is paramount in addressing customer needs. Also, different crops require different types of fertilizer having an appropriate S/N ratio. This requires fertilizers producers to be more flexible in the production of different urea ammonium sulphate qualities. The above referred to methods are, however, limited in controlling the S/N ratio of the final urea ammonium sulphate product to a desired predetermined value. The present invention aims to provide processes that allow for steering the quality of the obtained urea ammonium sulphate product more precisely and efficiently. It is thus an object of the present invention to provide a process and a plant that allows for the flexible production of urea ammonium sulphate grades having predetermined S/N ratios.

### SUMMARY OF THE INVENTION

The current invention provides in a solution for at least one of the above mentioned problems by providing a method for processing exhaust gases from a urea plant, the exhaust gas comprising urea, ammonia and/or ammonium sulphate.

In a first aspect, the present invention provides a method for recovery of urea dust and ammonia from a gas stream, being a waste air stream from granulation, prilling or product cooling process steps, comprising the steps of:
(i) contacting a gas stream, comprising urea dust and ammonia, with an aqueous sulphuric acid solution, thereby forming an acidic ammonium sulphate solution comprising urea,
(ii) neutralizing said acidic ammonium sulphate solution by adding ammonia, and
(iii) concentrating the neutralized solution obtained in neutralization step (ii) to a melt comprising less than 5 wt.% water, which melt is subsequently particulated into solid particles comprising urea and ammonium sulphate, whereby urea is added after neutralization step (ii) to obtain urea ammonium sulphate having a predetermined ratio of sulfur (S) atoms to nitrogen (N) atoms (S/N ratio).

As such, the inventors have realized that adding urea to urea ammonium sulphate allows to steer the S/N ratio much faster compared to adding ammonium sulphate to urea ammonium sulphate. In addition to a better quality control, the new methodology allows for a more energy efficient process.

In a second aspect, the present invention provides urea ammonium sulphate obtained by a method according to the first aspect of the invention.

### DESCRIPTION OF THE FIGURES

By means of further guidance, a figure is included to better appreciate the teaching of the present invention. Said figure is intended to assist the description of the invention and are nowhere intended as a limitation of the presently disclosed invention.

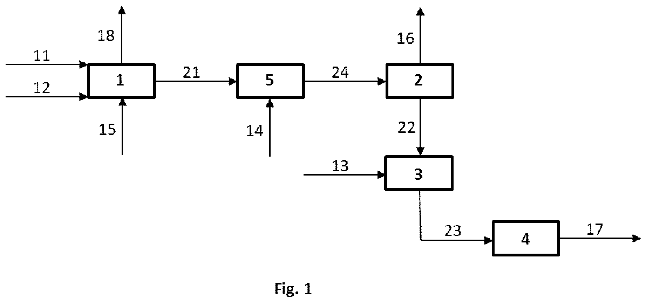

The figure and symbols contained therein have the meaning as commonly understood by one of ordinary skill in the art to which this invention belongs. Figure 1 shows a schematic system for implementing an embodiment of the present invention.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints. All percentages are to be understood as percentage by weight and are abbreviated as "%wt.", unless otherwise defined or unless a different meaning is obvious to the person skilled in the art from its use and in the context wherein it is used.

Preferably, the exhaust gas stream comprising urea dust and ammonia further comprises air. Hence the exhaust gas stream, as used in this application, is a stream of at least air, comprising at least nitrogen and oxygen in variable concentration. Preferably, the air is atmospheric air comprising a naturally occurring nitrogen and oxygen content, preferably comprising about 78 % nitrogen and about 21 % oxygen, more preferably comprising about 78 % nitrogen, about 21 % oxygen, about 0.9 % argon, about 0.04 % carbon dioxide, and small amounts of other gases.

The current invention provides in a solution for at least one of the above mentioned problems by providing a method for processing of urea and ammonia from exhaust gas from a urea plant.

The term "S/N ratio" as used within the context of the present disclosure is to be understood as the ratio of sulphur (S) atoms to nitrogen (N) atoms within a mixture, i.e. a urea ammonium sulphate mixture.

The term "urea-free ammonium sulphate" as used within the context of the present disclosure is to be understood as a liquid or solid ammonium sulphate having less than 10 wt.% urea, preferably less than 5 wt.% urea, more preferably less than 1 wt.% urea and most preferably less than 0.5 wt.% urea.

In a first aspect, the present invention provides a method for recovery of urea dust and ammonia from a gas stream, being a waste air stream from granulation, prilling or product cooling process steps in a urea plant, comprising the steps of:
(i) contacting said gas stream, comprising urea dust and ammonia, with an aqueous sulphuric acid solution, thereby forming an acidic ammonium sulphate solution comprising urea,
(ii) neutralizing said acidic ammonium sulphate solution by adding ammonia, and
(iii) concentrating the neutralized solution obtained in neutralization step (ii) to a melt comprising less than 5 wt.% water, which melt is subsequently particulated into solid particles comprising urea and ammonium sulphate,
whereby urea is added after neutralization step (ii) to obtain urea ammonium sulphate having a predetermined S/N ratio. More specifically, urea is added after neutralization step (ii) to urea-free, neutralized ammonium sulphate, preferably in solution, to obtain urea ammonium sulphate having a predetermined S/N ratio.

Particular examples of urea ammonium sulphate having distinct qualities as characterized by a distinct S/N ratio are YaraVera™ UREAS and YaraVera™ AMIDAS, both grades produced by Yara International ASA. AMIDAS has an S/N ratio of 1:7.3 whereas UREAS has an S/N ratio of 1:5. Urea comprises two atoms of nitrogen and does not comprise sulphur. In contrast, ammonium sulphate comprises two parts of nitrogen for every part of sulphur. As such, the inventors have found that adding urea to urea ammonium sulphate allows to steer the S/N ratio much faster compared to adding ammonium sulphate to urea ammonium sulphate. Importantly, this allows the production of urea ammonium sulphate with a predetermined S/N ratio to the production of urea ammonium sulphate with a different, predetermined S/N ratio in an efficient manner. In addition to a better production control, the new methodology allows for a more energy efficient process.

In a preferred embodiment, the present invention provides a method according to the first aspect of the invention, wherein urea is mixed into said melt prior to particulating into solid particles. This allows for a homogeneous urea ammonium sulphate mixture after particulating into solid particles, and thereby avoiding that urea particles and ammonia particles shift during transporting or during storage of the urea ammonium sulphate particles.

In a preferred embodiment, the present invention provides a method according to the first aspect of the invention, wherein urea is mixed into the neutralized solution obtained in neutralization step (ii) prior to concentrating the neutralized solution. Mixing urea in an aqueous solution allows for an easier, and thus less energy intensive, mixing compared to mixing with a more viscous melt.

In a preferred embodiment, the present invention provides a method according to the first aspect of the invention, wherein urea is added as an aqueous urea solution. Preferably, said aqueous urea solution comprises urea in a concentration of more than 10 wt.%, and preferably in a concentration of more than 20 wt.%, more than 30 wt.% and even more than 50 wt.%. As such, the aqueous urea solution is a highly concentrated urea solution. In an alternative or further embodiment, the aqueous urea solution is an urea slurry. In a more preferred embodiment, the present invention provides a method according to the first aspect of the invention, wherein urea is added as a solid. This allows to affect the S/N ratio of the final urea ammonium sulphate particulate quickly, and accordingly to provide the correct quality of the fertilizer product.

In a preferred embodiment, the present invention provides a method according to the first aspect of the invention, wherein urea dust and ammonia are separated prior to step (i). Such an embodiment provides a more energy efficient manner of treating ammonia in the process exhaust gas stream. Furthermore, this allows to avoid direct contact between sulphuric acid and urea.

In a preferred embodiment, the present invention provides a method according to the first aspect of the invention, wherein ammonia is added in said neutralization step (ii) in a ratio of about 1:1 to the excess of sulphuric acid in said acidic ammonium sulphate solution. Neutralization to a 1:1 ratio avoids the presence of ammonia and sulphuric acid in the obtained neutralized solution and ensures that all components are enclosed in the resulting product stream as an ammonium sulphate salt which is a productive fertilizer.

In a preferred embodiment, the present invention provides a method according to the first aspect of the invention, wherein said gas stream further comprises ammonium sulphate dust. Urea ammonium sulphate dust and/or ammonium sulphate dust may be captured from a granulator, a prilling tower, or a pelletizer, comprising a feeding device, a solidification/cooling belt and a device to remove the formed pellets from the belt, by feeding a urea- and/or ammonium sulphate-comprising liquid to the feeding device from which droplets of the liquid are dosed to the belt, whereon the droplets solidify, where after the formed particles are removed from the belt. The belt is cooled from the other side, preferably by means of cooling water.

In a preferred embodiment, the present invention provides a method according to the first aspect of the invention, wherein said neutralized solution is concentrated by vaporization. This is advantageous to remove any volatile products such as sulphuric acid, ammonia, etc. which may be present in the ammonium sulphate solution. More preferably, the vaporization is carried out in more than one step until the amount of water is less than 5 wt.%. This allows reducing the amount of water in the melt to less than 1 wt.%, and even to less than 0.3 wt.%.

In a preferred embodiment, the present invention provides a method according to the first aspect of the invention, wherein the vapour obtained in the concentration step (iii) is subsequently condensed, thereby forming an aqueous phase. Preferably, the aqueous phase - which may further comprise process condensates such as sulphuric acid, ammonia, etc. - is used as process water within the process. As such, any N- and/or S-compounds can efficiently be recycled.

In a preferred embodiment, the present invention provides a method according to the first aspect of the invention, wherein ammonium sulphate is added after neutralization step (ii) to obtain urea ammonium sulphate having a predetermined S/N ratio.

A system for implementing the method of the invention is shown in Fig. 1. A gas stream comprising air, urea and ammonia **11** is contacted in a scrubber **1** with an aqueous sulphuric acid solution **12,** thus forming an acidic solution of urea and ammonium sulphate.

Scrubber **1** can be selected from any of the wet-type scrubbers well known in the industry. It may, for instance, be selected from the type of scrubbers as summarized in Chemical Engineers Handbook (Perry and Chilton), fifth edition, page 20-94 to 20-103. Stream **11** usually has a relative high temperature (70°C to 110°C), and may be rather dry. As a result of this, quite some water may evaporate in the scrubber. In many cases it therefore will be required to add make-up water **(15)** to the scrubber in order to assure that the concentration of urea and ammonium sulphate in the liquid phase in the scrubber remains below the solubility limits. Depending on the type of scrubber selected, circulation of acidic urea ammonium sulphate solution over the scrubber (not shown in the figure) may be required for proper removal of ammonia and dust from the air stream. The cleaned air leaves the scrubber via line **18.**

The acidic solution of urea and ammonium sulphate is passed through line **21** to concentration unit **2,** which may comprise at least one concentrator. Water vapour leaves the concentration unit **2** via line **16.** In the concentration unit **2,** the acidic solution of urea and ammonia is concentrated to a melt, comprising less than 5 wt.% of water. The concentration unit **2** may consist of one or more evaporators in parallel or in series. These evaporators may be selected from evaporators, as they are well known in the process industry. They may, for instance, be selected from the evaporators as summarized in Chemical Engineers Handbook (Perry and Chilton), fifth edition. Urea is vulnerable for decomposition (e.g. hydrolyses and biuret formation) at high temperatures and at long residence time. For this reason, the evaporators are usually selected from the types 'falling film' or 'Long tube vertical' (refer to Figs. 11-16 in Chemical Engineers Handbook (Perry and Chilton), fifth edition) since they offer low residence time. Also, in order to minimize urea decomposition, the evaporators preferably are operated under vacuum, in order to minimize the required temperature. The vacuum in the evaporators can be maintained using a system of vacuum condensers and steam-jet ejectors (not shown in the figure), or other systems, that are well known in the industry.

The concentrated urea ammonium sulphate melt leaves the concentration unit via line **22** to mixer **3.** A highly concentrated urea solution **13** is also introduced into the mixer **3,** in order to increase the ammonium sulphate to urea ratio to the desired value. The dosing of urea to mixer **3** is controlled in such a way that a stable S/N ratio is obtained in the final product **17.** Mixer **3** may be selected from any of the solid/liquid mixers well known in the industry. It may e.g. be selected from the mixers as summarized in Chemical Engineers Handbook (Perry and Chilton), fifth edition, pages 19-3 to 19-25. Selection of the mixer mainly is depending on the required ammonium sulphate to urea ratio. In case low concentrations of ammonium sulphate are required, then the solid concentration in the urea ammonium sulphate slurry **23** will be low. In that case, it will be sufficient to select the mixer from the class of 'agitating mixers'. In case higher concentrations of ammonium sulphate are required, then the mixer more effectively can be selected of the class of 'paste and viscous material mixing' equipment. From the mixer, the slurry of solid ammonium sulphate in a urea ammonium sulphate melt is transported via line **23** to the solid shaping step **4.**

The solid shaping step **4** may consist of granulation, prilling or pelletizing. It is of special advantage to select pelletizing as solid shaping process, since such a pelletizing process does not result in dust and ammonia loaded off-gas as is the case with prilling and granulation processes. An example of such a pelletizing process is described in WO 2006/111331 A1. The final product, a solid mixture of urea and ammonium sulphate, leaves the process via line **17.**

Fig. 1 further shows a system for implementing the method of the invention, wherein ammonia or ammonia water **14** is added to the acid solution in a neutralizer **5** before the acidic solution **21** is concentrated, thus forming a neutralized solution of urea and ammonium sulphate in water that is passed through line **24** to the concentration unit **2.** The neutralizing process **5,** may be accommodated in a mixing vessel with agitator, as well known in the industry. Taking into account the strong chemical affinity between sulphuric acid and ammonia, the neutralizing process may even be accommodated in a much simpler way, e.g. by supplying turbulent flow and sufficient residence time in the process line that transports urea ammonium sulphate solution **24** to the concentration unit **2.**

### List of reference numbers

- 1: Scrubber
- 2: Concentration unit
- 3: Mixer
- 4: Solid shaping step (granulation, etc.)
- 5: Neutralizer
- 11: Gas stream comprising air, urea and ammonia
- 12: Sulphuric acid solution
- 13: Concentrated urea solution
- 14: Ammonia
- 15: Water
- 16: Water vapour
- 17: Final product
- 18: Cleaned air
- 21: Acidic solution of urea and ammonium sulphate
- 22: Concentrated urea ammonium sulphate melt
- 23: Urea ammonium sulphate slurry
- 24: Urea ammonium sulphate solution

## Claims

1. A method for recovery of urea dust and ammonia from a gas stream, being a waste air stream from granulation, prilling or product cooling process steps in a urea plant, comprising the steps of:
(i) contacting a gas stream, comprising urea dust and ammonia, with an aqueous sulphuric acid solution, thereby forming an acidic ammonium sulphate solution comprising urea,
(ii) neutralizing said acidic ammonium sulphate solution by adding ammonia, and
(iii) concentrating the neutralized solution obtained in neutralization step (ii) to a melt comprising less than 5 wt.% water, which melt is subsequently particulated into solid particles comprising urea and ammonium sulphate,
**characterized, in that** urea is added after neutralization step (ii) to obtain urea ammonium sulphate having a predetermined ratio of sulphur (S) atoms to nitrogen (N) atoms (S/N ratio).

2. Method according to claim 1, wherein urea is mixed into said melt prior to particulating into solid particles.

3. Method according to any one of claims 1 to 2, wherein urea is mixed into said neutralized solution obtained in neutralization step (ii) prior to concentrating the neutralized solution.

4. Method according to any one of claims 1 to 3, wherein urea is added as an aqueous urea solution.

5. Method according to any one of claims 1 to 4, wherein urea is added as a solid.

6. Method according to any one of claims 1 to 5, wherein ammonia is added in said neutralization step (ii) in a ratio of about 1:1 to the excess of sulphuric acid in said acidic ammonium sulphate solution.

7. Method according to any one of claims 1 to 6, wherein said gas stream further comprises ammonium sulphate.

8. Method according to any one of claims 1 to 7, wherein said neutralized solution is concentrated by vaporization.

9. Method according to claim 8, wherein the vapour obtained in concentration step (iii) is subsequently condensed, thereby forming an aqueous phase.

10. Method according to any one of claims 1 to 9, wherein ammonium sulphate is added after neutralization step (ii) to obtain urea ammonium sulphate having a predetermined ratio of sulphur (S) atoms to nitrogen (N) atoms (S/N ratio).

## Patentansprüche

1. Verfahren zur Gewinnung von Harnstoffstaub und Ammoniak aus einem Gasstrom, bei dem es sich um einen Abluftstrom aus Prozessschritten der Granulierung, dem Prillen oder der Produktkühlung in einer Harnstoffanlage handelt, umfassend die Schritte:
(i) Inkontaktbringen eines Harnstoffstaub und Ammoniak umfassenden Gasstroms mit einer wässrigen Schwefelsäurelösung, wodurch eine Harnstoff umfassende saure Ammoniumsulfatlösung gebildet wird,
(ii) Neutralisieren der sauren Ammoniumsulfatlösung durch Zugeben von Ammoniak und
(iii) Aufkonzentrieren der im Neutralisierungsschritt (ii) erhaltenen neutralisierten Lösung zu einer weniger als 5 Gew.-% Wasser umfassenden Schmelze, wobei die Schmelze anschließend zu Harnstoff und Ammoniumsulfat umfassenden festen Partikeln partikuliert wird,
**dadurch gekennzeichnet, dass** Harnstoff nach dem Neutralisierungsschritt (ii) zum Erhalt von Harnstoffammoniumsulfat mit einem vorgegebenen Verhältnis von Schwefel(S)-Atomen zu Stickstoff(N)-Atomen (S/N-Verhältnis) zugegeben wird.

2. Verfahren nach Anspruch 1, wobei Harnstoff der Schmelze vor dem Partikulieren zu festen Partikeln zugemischt wird.

3. Verfahren nach einem der Ansprüche 1 bis 2, wobei Harnstoff der im Neutralisierungsschritt (ii) erhaltenen neutralisierten Lösung vor dem Aufkonzentrieren der neutralisierten Lösung zugemischt wird.

4. Verfahren nach einem der Ansprüche 1 bis 3, wobei Harnstoff als wässrige Harnstofflösung zugegeben wird.

5. Verfahren nach einem der Ansprüche 1 bis 4, wobei Harnstoff als Feststoff zugegeben wird.

6. Verfahren nach einem der Ansprüche 1 bis 5, wobei Ammoniak im Neutralisierungsschritt (ii) in einem Verhältnis von etwa 1:1 zu der überschüssigen Schwefelsäure in der sauren Ammoniumsulfatlösung zugegeben wird.

7. Verfahren nach einem der Ansprüche 1 bis 6, wobei der Gasstrom ferner Ammoniumsulfat umfasst.

8. Verfahren nach einem der Ansprüche 1 bis 7, wobei die neutralisierte Lösung durch Verdampfen aufkonzentriert wird.

9. Verfahren nach Anspruch 8, wobei der im Aufkonzentrierungsschritt (iii) erhaltene Dampf anschließend kondensiert wird, wodurch eine wässrige Phase gebildet wird.

10. Verfahren nach einem der Ansprüche 1 bis 9, wobei Ammoniumsulfat nach dem Neutralisierungsschritt (ii) zum Erhalt von Harnstoffammoniumsulfat mit einem vorgegebenen Verhältnis von Schwefel(S)-Atomen zu Stickstoff(N)-Atomen (S/N-Verhältnis) zugegeben wird.

## Revendications

1. Procédé pour la récupération de poussière d'urée et d'ammoniac d'un flux de gaz, qui est un flux d'air d'évacuation provenant d'étapes de procédé de granulation, de grelonage ou de refroidissement de produits dans une installation d'urée, comprenant les étapes de :
(i) mise en contact d'un flux de gaz, comprenant de la poussière d'urée et de l'ammoniac, avec une solution aqueuse d'acide sulfurique, formant ainsi une solution de sulfate d'ammonium acide comprenant de l'urée,
(ii) neutralisation de ladite solution de sulfate d'ammonium acide par ajout d'ammoniac, et
(iii) concentration de la solution neutralisée obtenue dans l'étape de neutralisation (ii) pour donner une masse fondue comprenant moins de 5 % d'eau, laquelle masse fondue est subséquemment transformée en particules solides comprenant de l'urée et du sulfate d'ammonium,
**caractérisé en ce que** de l'urée est ajoutée après l'étape de neutralisation (ii) pour obtenir du sulfate d'ammonium urée possédant un rapport prédéterminé d'atomes de soufre (S) sur les atomes d'azote (N) (rapport S/N).

2. Procédé selon la revendication 1, de l'urée étant mélangée dans ladite masse fondue avant sa transformation en particules solides.

3. Procédé selon l'une quelconque des revendications 1 et 2, de l'urée étant mélangée dans ladite solution neutralisée obtenue dans l'étape de neutralisation (ii) avant la concentration de la solution neutralisée.

4. Procédé selon l'une quelconque des revendications 1 à 3, de l'urée étant ajoutée en tant que solution aqueuse d'urée.

5. Procédé selon l'une quelconque des revendications 1 à 4, de l'urée étant ajoutée en tant qu'un solide.

6. Procédé selon l'une quelconque des revendications 1 à 5, de l'ammoniac étant ajouté dans ladite étape de neutralisation (ii) en un rapport d'environ 1 : 1 par rapport à l'excès d'acide sulfurique dans ladite solution de sulfate d'ammonium acide.

7. Procédé selon l'une quelconque des revendications 1 à 6, ledit flux de gaz comprenant en outre du sulfate d'ammonium.

8. Procédé selon l'une quelconque des revendications 1 à 7, ladite solution neutralisée étant concentrée par vaporisation.

9. Procédé selon la revendication 8, la vapeur obtenue dans l'étape de concentration (iii) étant subséquemment condensée, formant ainsi une phase aqueuse.

10. Procédé selon l'une quelconque des revendications 1 à 9, du sulfate d'ammonium étant ajouté après l'étape de neutralisation (ii) pour obtenir du sulfate d'ammonium urée possédant un rapport prédéterminé d'atomes de soufre (S) sur les atomes d'azote (N) (rapport S/N).
